# EUROPEAN PATENT SPECIFICATION

(11) **EP 0 642 794 B1**
(45) Date of publication and mention of the grant of the patent: **04.08.1999**
(21) Application number: 94114383.6
(22) Date of filing: 13.09.1994
(51) Int. Cl.: A61K 35/78

(54) **Pharmaceutical composition for prophylaxis and treatment of premature ejaculation**
Arzneimittelzusammensetzung zur Prophylaxe und Behandlung vorzeitigen Samenergusses
Composition pharmaceutique pour la prophylaxie et le traitement de l'éjaculation précoce

(30) Priority: 14.09.1993 KR 9318467
(43) Date of publication of application: 15.03.1995
(73) Proprietor: Choi, Hyung Ki, Youngdeungpo-gu, Seoul (KR); Xin, Zhong Cheng, JiLin City, JiLin Province (CN)
(72) Inventor: Choi, Hyung Ki, Youngdeungpo-gu, Seoul (KR); Xin, Zhong Cheng, JiLin City, JiLin Province (CN)
(74) Representative: Dost, Wolfgang, Dr.rer.nat., Dipl.-Chem.

(56) References cited:
- EP-A- 0 415 613
- DE-A- 2 405 115

## Description

### 1. Field of the invention

The present invention relates to a pharmaceutical composition for the prophylaxis and treatment of premature ejaculation. More particularly, the present invention relates to a galenic composition for the prophylaxis and treatment of male ejaculation accommodation ataxia due to sexual nervous hypersensitivity and to a process for preparing said galenic composition.

### 2. Background art

The nervous system of the human body can be classified into the central nervous system and the peripheral nervous system. Nervous hypersensitivity (neuroticism) is generally caused by a disorder of the physico-neurological system which is under the influence of the central nervous system. However, sexual nervous hypersensitivity is mainly caused by a disorder in the complex cooperation between the peripheral nervous system and the central nervous system. Meanwhile, with the advance of modern civilization and the development of scientific technique an activation of the central nervous system is preferentially necessary for achieving high mental activities, such as the adaptation to novel scientific techniques, maintenance of smooth human relations and to maintain social discipline rather than to pursue the instinctive physical satisfaction in, for example, the necessities of life and sexual desire.

Such complicated social life results in a destruction of the balance and smooth cooperation of central nervous system and peripheral nervous system. Particularly, in men, this may cause a loss of the ability to achieve sexual accommodation which is necessary for the satisfaction of the human instinctive desire. Recently, it has been determined that the number of cases manifesting various symptoms caused by such loss of sexual accommodation is rather large.

According to statistics, it has been shown that although there is some differences between age groups, approximately 30 to 50% of Korean adult men visiting to impotence clinic suffer from sexual hypersensitivity. The typical example of sexual hypersensitivity is premature ejaculation, which is the main reason for sexual problems and which leads to social difficulties, such as asthenia due to the loss of self-confidence, as well as domestic discord. Premature ejaculation is defined as persistent or recurrent ejaculation before, upon, or shortly after penetration.

The reasons for premature ejaculation is generally thought to include a malfunction of the repressor center due to the fatigue of nervous transmission, hypersensitivity of a specific site due to genital disorders, hormonal disorders, physical problems and the like. It is believed that the premature ejaculation is generally caused by a complex interaction of the above mentioned reasons or by a loss of cooperation among the related sexual nerve centers.

Premature ejaculation has been treated with psychotherapy and drug therapy. Psychotherapy requires sexual training for a long period of time which involves discussions and cooperation with a physician and the patient and his wife. However, since psychotherapy necessitates a long period of time for the doctor, patient and his wife to work together in order to be effective its success rate is low. That is, changes in living style, external stress, etc., undermine its success such that the problem either never is solved or it reoccurs. Therefore, drug therapy is now more widely used since time restrictions are not as great.

Present drug therapy includes psychotropic agents which suppress the excitation of sexual nerve center and local anesthetic for blunting the sensitivity of the sexual peripheral nerve. However, since the central nervous system depressants may make the sexual act itself impossible due to a loss of sexual desire and since local anesthetic, such as lidocaine ointment or spray, sometimes induces vasoconstriction which may lead to transient erectile failure, and should be applied just before the sexual act because of its short duration, present day drug therapy cannot successfully solve the problems concerned with premature ejaculation.

Thus, the present inventors have extensively investigated the available literature and have continuously conducted studies and experiments related overcoming the problem of premature ejaculation. As a result, the inventors have found that the suitable combination of certain galenic substances can provide an excellent pharmacological effect to overcome premature ejaculation without any of the problems associated with the prior therapeutic methods or drugs as mentioned above.

Therefore, it is an object of the present invention to provide a pharmaceutical galenic composition for the prophylaxis and treatment of premature ejaculation.

A further object of the present invention is to provide a pharmaceutical composition suitable for prophylaxis and treatment of premature ejaculation, which contains effective amounts of : extracts of ginseng radix, angelicae gigantis radix, broomrape, cassiae cortex, asiasari radix and bufonis venenum as the essential components.

A further object of the present invention is to provide a pharmaceutical composition for the prophylaxis and treatment of sexual hypersensitivity, which further contains one or more additional galenic substance selected from the group consisting of: extracts of xanthoxyli fructus, cnidium fructus, caryophylli flos and moschus, in addition to effective amounts of: extracts of ginseng radix, angelicae gigantis radix, broomrape, cassiae cortex, asiasari radix and bufonis venenum as the essential components.

A further object of the present invention is to provide a process for preparation of said pharmaceutical composition for the prophylaxis and treatment of premature ejaculation.

### DISCLOSURE OF INVENTION

In the pharmaceutical composition according to the present invention ginseng radix, angelicae gigantis radix, broomrape, cassiae cortex, asiasari radix and bufonis venenum are used as the essential galenic components. In addition to the above essential components, the pharmaceutical composition of the present invention can preferably contain one or more additional galenic components selected from the group consisting of: moschus, xanthoxyli fructus, cnidium fructus and caryophylli flos.

Each of the raw galenic components used in the present invention exhibit certain pharmacological effects. Ginseng radix is used for its tonic, analeptic and sedative activities and for its fatigue amelioration and its effect of stimulating metabolism. Angelicae gigantis radix is generally used in the treatment of anemia and blood flow disorders in obstetrics and in gynecology for the purpose of blood supplement and pain relief. Broomrape is effective for the enhancement of sexual desire and as an analeptic. In addition, cassiae cortex contains an essence component in a large amount, which exhibits peripheral vasodilating activity and also the stimulating effect in a small amount, but the inhibiting effect in a large amount, on the central nervous system, and is generally used as an analgesic, anticonvulsant and tonic agent. Asiasari radix contains an essence component which exhibits antihistaminic effect and, therefore, is clinically used as an antipyretic, analgesic, antitussive and expectorant. Bufonis venenum is a substance secreted from the otic gland of toads which has cardiotonic, analeptic, diuretic, analgesic and detoxicating effects and also has a superior local anesthetic effect and vasodilating effect. In addition, xanthoxyli fructus has topical sensory paralysis activity and peripheral vasodilating activity and is clinically used as an analgesic and anticonvulsant. Moschus is a dry substance of musk pod secretions from musk deer and is widely used as analeptic, cardiotonic, tonic, sedative, anticonvulsant, pusremoving and detoxicating agents in view of its tonic, analeptic, anti-inflammatory and beta-adrenergic effects. Further, both cnidium fructus and caryophylli flos possess analeptic and peripheral vasodilating effects.

The pharmaceutical composition of the present invention can be formulated in the form of ointments, pills, suspensions, gels or liquid formulations for topical application by the process which comprises either extracting with a solvent or pulverizing the galenic substances depending on physico-chemical properties of their active components, combining the obtained extracts and/or powders and then formulating the mixture into the desired formulations according to conventional methods known in the art of pharmacy.

Although the mechanism of pharmacological action of the composition of the present invention cannot be exactly identified, it is believed that the analgesic, sedative and anesthetic action of the composition of the present invention may reduce or paralyze the sensory function of a hypersensitive glans receptor, which is the main portion of the ejaculation reflex nerve, and block the transfer pathway to the central nervous system to sedate the (sexual) exciting center and at the same time the blood flow accelerating activity of the present composition may cause the erection to isolate the ejaculation reflex nerve from the sexual contact surface so that the hypersensitive sexual nerve cannot be stimulated thereby preventing premature ejaculation. The above explanation is merely provided as one explanation of how the composition according to the present invention functions. However, the inventors do not want to be held to this explanation as being the only explanation of the mechanism of action.

More specifically, according to the present invention 60-140 parts by weight of ginseng radix, 60-140 parts by weight of angelicae gigantis radix, 60-140 parts by weight of broomrape, 10-30 parts by weight of cassiae cortex, 10-30 parts by weight of asiasari radix and 15-25 parts by weight of bufonis venenum as essential components can be appropriately treated and combined to prepare the desired pharmaceutical composition. Preferably, one or more additional galenic component selected from the group consisting of: 40-70 parts by weight of xanthoxyli fructus, 60-140 parts by weight of cnidium fructus, 10-30 parts by weight of caryophylli flos and 5-15 parts by weight of moschus can be further incorporated into the pharmaceutical composition of the present invention.

The constitutional ratio of the galenic components as described above was established on the basis of the result obtained from numerous animal experiments. When the galenic components are combined in the ratio lower than the lowest level as defined above, the pharmacological effect of the corresponding components was drastically reduced. On the other hand, when any component is combined in the ratio higher than the highest level thereof, the effect of other component was reduced and the synergistic and cooperative action of the composition of the present invention is significantly reduced. Therefore, it is critical that the galenic components of the composition of the present invention are combined in the ratio as defined above.

Among the raw galenic substances, ginseng radix, angelicae gigantis radix, broomrape, cnidium fructus and xanthoxyli fructus are used in the form of extracts obtained by extraction of the active components with alcohol or water. Cassiae cortex, caryophylli flos and asiasari radix are used by immersion in water and then concentrated. Bufonis venenum is used either in the form of a powder obtained by dissolving it in human milk and then concentrating it to dryness or in the form of the extract obtained by extraction of the active component with alcohol or water. Moschus is prepared as a dry powder or the alcohol extract. Said pre-treated galenic substances can be uniformly mixed with pharmaceutically acceptable carriers to obtain the desired pharmaceutical composition for treating premature ejaculation according to the present invention.

In view of the nature of the indication, the composition of the present invention is most preferably administered in the form of a topical ointment. However, if necessary, the composition of the present invention can be prepared and stored in the form of a gel, suspension, solution, dispersion, spray or pill or in the form of a tablet, which may be converted into another formulation when the composition is used.

If the composition of the present invention is in the form of an ointment, it is preferable that about 0.1-0.5g of the ointment be externally applied to a portion of glans penis 30 minutes to 10 hours before the expected initiation of sexual intercourse. Then in about 20 to 30 minutes after application the ointment may be washed off with water in order to remove any unpleasant feeling that may accompany the topical application. In 20 to 30 minutes after topical application genital hypersensitivity is dulled or blunted and blood flow is stimulated. The effect of the ointment lasts for about 8 to 12 hours.

The present invention will be more specifically illustrated by the following example and experiments. However, it should be understood that the present invention is not limited by these examples in any manner.

### EXAMPLE 1 : Ointment Preparation

a) 100g of ginseng radix, 100g of angelicae gigantis radix, 100g of broomrape, 100g of cnidium fructus and 50g of xanthoxyli fructus were washed with water, dried, ground, and then immersed in 2500ml of 90% ethanol for 24 hours and filtered. The residue was again immersed in 2000ml of 90% ethanol for 24 hours and filtered. The obtained two filtrates were combined and distilled to remove ethanol and to obtain the extract. The residue from the above ethanol extraction was immersed in 2500ml of water for 4 hours at the temperature below 80^{o}C. The extract was then filtered to remove the residue. The filtrate was immersed in 2500ml of 95% ethanol for 24 hours under cooling. The mixture was filtered to remove the precipitated substance. The filtrate was distilled to remove ethanol and then combined with the alcohol extract obtained above. The combined extract was concentrated under reduced pressure to 1000ml, and 10g of active carbon was added thereto. The mixture was warmed to 80^{o}C for 30 minutes and then filtered. The filtrate was concentrated at the temperature below 80^{o}C under reduced pressure and then lyophilized to obtain about 100g of the extract powder.
b) 20g of cassiae cortex, 20g of caryophylli flos and 20g of asiasari radix were cleaned by washing with water, immersed in 300ml of distilled water for 4 hours and then doubly distilled to obtain about 5g of the extract comprising the essence components.
c) 20g of bufonis venenum was immersed in 1000ml of 70% ethanol at 50^{o}C for 4 hours and then filtered. The filtrate was distilled to remove ethanol. The filter cake was immersed again in 500ml of distilled water at 50^{o}C for 4 hours and then filtered. The filtrate was combined with the previously obtained filtrate, and 5g of active carbon was added thereto. The mixture was warmed at 80^{o}C for 30 minutes and filtered. The filtrate was concentrated under reduced pressure at the temperature below 50^{o}C and then lyophilized to obtain 5g of a powder.
d) 10g of moschus was immersed in 500ml of 70% ethanol at 50^{o}C for 4 hours and filtered. The filtrate was distilled to remove ethanol. The filter cake was immersed again in 300ml of distilled water at 50^{o}C for 4 hours and then filtered. The filtrate was combined with the previously obtained filtrate, and 3g of active carbon was added thereto. The mixture was warmed to 80^{o}C for 30 minutes and filtered. The filtrate was concentrated under reduced pressure at the temperature below 50^{o}C and then lyophilized to obtain 2g of a powder.

The powders obtained in a), c) and d) above were mixed uniformly with the essence obtained in b) above. Then the mixture was uniformly mixed with polyethylene glycol as water-soluble base in the ratio of 1:9 by weight to prepare a yellow colored ointment composition according to the present invention.

### EXPERIMENT 1 : Test for toxicity and side effects

a) About 0.2g of the ointment prepared in above EXAMPLE 1 was applied five (5) times at the interval of 10 minutes to glans penis of each of 5 adult male rabbits and then the appearance of the penis was observed every hour for 12 hours. Then the ointment was washed off the penis of each rabbit and the appearance of penis was observed every 30 minutes for 10 hours. As a result, a hyperemia due to the genital vasodilation continuously remained from one hour after the ointment of the present invention was applied and then remained for about 10 hours even after removal of the ointment. Thereafter, the hyperemia gradually disappeared. However, the application of the ointment of the present invention did not cause any denaturation of the genital epidermis or any histological change and also, no functional disorder was observed.
b) The present inventors have determined a suitable dosage and a method of use by topically applying the ointment according to the present invention to themselves on several different times. As a result of this, the dosage for single application to obtain the desired effect is about 0.1 to 0.2g of the ointment applied to the glans of the penis. The ointment was applied three times with 0.2g of the ointment being applied at each application at an interval of 30 minutes apart. The ointment was allowed to remain on for 10 hours before being washed off. As a result, the topical or systemic side effect, except for a topical burning sensation due to vasodilation, could not be identified. Said topical burning sensation disappeared after 30 minutes in accordance with the blunting of the sense in glans portion of the penis.

### EXPERIMENT 2 : Clinical test

a) This clinical test was conducted for 30 patients with an informed consent, who suffer from serious sexual disorder due to premature ejaculation and who agreed with this clinical trial schedules at the Yongdong Severance Hospital, Urology Department, residing in Kangnam-ku, Seoul, Korea, under surveillance of the present inventors with the ointment prepared in the above EXAMPLE 1 according to the present invention. The age of the subject patients was in the range of 32 to 57 and the lasting period of the sexual functional disorder was 3 months to 30 years. Those patients were divided into 15 patients suffering from simple premature ejaculation and 15 patients suffering from premature ejaculation and accompanying erection failure. All of the subject patients did not show any hormonal disorder and any abnormality in physico-chemical examination. According to the audio-visual stimulating test (AVS-Penogram) using a radioisotope (Technetium^{99m}-RBC), the patients were classified into the group of 14 patients showing normal dynamic change in penis blood flow (Type I) and the group of 16 patients showing abnormal change in penis blood flow (Type II). The main etiological factor in those patients was determined as psychogenic cause. The subject patients included 2 patients who have never improved their premature ejaculation even after implantation of penile prosthesis and 20 patients who did not show any effect with a commercial local anesthetic spray.
   The patient was instructed to uniformly apply the ointment of the present invention to penis glans in an amount of 0.2g for each time sexual intercourse was anticipated. Then the ointment was to be washed off after 20 to 30 minutes. Thereafter, the patients were advised to have sexual intercourse within 2 to 8 hours and report the ejaculation accomodation ability every week. The ejaculation accommodation ability of the ointment of the present invention was estimated by examining the change in time from the initiation of sexual act to the ejaculation and the conjugal satisfaction feeling through 4 times of monitoring at the interval of one week. As a result, in the patients having simple premature ejaculation the ejaculation time was extended from not more than 2 minutes before the use of the ointment according to the present invention, to 15 to 20 minutes after the use of the ointment. In the group of the patients suffering from erection failure and premature ejaculation it was reported that as well as premature ejaculation, the erection state was somethat improved. Before use of the ointment, 13 severe patients ejaculated just before or just after vaginal penetration, 12 patients ejaculated within one minute just after penetration and 5 patients ejaculated within 2 minutes after penetration. However, at the time of first report after one week from use of the ointment, the delay in ejaculation time increased to 10 minutes in 2 patients, 15 minutes in 13 patients and more than 20 minutes in 13 patients. Two (2) patients, including one suffering from chronic prostatitis, show little increase in the delay in ejaculation time from just after the penetration to 2 minutes and 5 minutes, respectively. In addition, at the time of third report it was examined that the ejaculation time was extended to 15 minutes in 2 patients and 20 minutes in 26 patients, except for the non-affected 2 patients. Thereafter, 11 of the above patients reported that a satisfactory sexual act is possible with-out using the ointment of the present invention, but the remaining patients could not be monitored any longer.
b) The effect of the composition of the present invention was compared with that of a commercial lidocaine cream. This comparative test was conducted for 50 volunteer patients, with the average age of 41.4, who suffer from premature ejaculation due to genital hypersensitivity and visit the Yongdong Severance Hospital. In this test, the ointment prepared in EXAMPLE 1 of the present invention, 1.5% lidocaine cream, 5% lidocaine cream, 10% lidocaine cream and a placebo were designated SS, LL, ML, HL and PB, respectively, so that the patients cannot know the used drug. The patients were advised to externally apply 5 kinds of the drugs, in order, to penis and then have sexual intercourse. That is, each of the lidocaine creams and the placebo was applied in an amount of 0.2g before 30 minutes to one hour from sexual intercourse and the ointment according to the present invention was applied in an amount of 0.2g before 1 to 8 hours from sexual intercourse. Then each of the tested drugs was washed off after 30 minutes. Thereafter, the patients were instructed to report the lasting time of sexual intercourse and the sex satisfaction after one month. Among the 50 volunteer patients, 43 patients submitted the reports within one to three months after the beginning of the test. The average value of the obtained results is described in the following table.

| Tested drug | Lasting time of sexual intercourse (min.) | Sex satisfaction⁽¹⁾ (number of patients) | | | | Remarks |
|---|---|---|---|---|---|---|
| | | A | B | C | D | |
| Before use | 0.8 | 43 | 0 | 0 | 0 | |
| PB | 1.3 | 40 | 3 | 0 | 0 | |
| LL | 1.9 | 38 | 5 | 0 | 0 | |
| ML | 2.2 | 36 | 7 | 0 | 0 | |
| HL | 3.7 | 30 | 8 | 3 | 2 | (2) |
| SS | 11.3 | 2 | 4 | 25 | 12 | |

| | | | | | | |
|---|---|---|---|---|---|---|
| Note : (1) A = dissatisfaction B = usual C = satisfaction D = very satisfaction | | | | | | |
| (2) 11 patients suffer from genital insensability and erection disorder. | | | | | | |

### EXPERIMENT 3 : Test for effect of stimulating the blood circulation

The temperatures of penis and scrotum of 10 normal volunteers were measured by means of Computerized Infrared Thermography Imaging. The ointment prepared in the above EXAMPLE 1 according to the present invention was applied to penis of each volunteer in an amount of 0.2g and then the temperatures of penis and scrotum were measured for 8 hours at the interval of 2 hours by means of the same method. As a result, it could be identified that the topical temperature of penis and scrotum continuously increased by about 0.8-1.1^{o}C from the average of 27.5^{o}C before use of the ointment to the average of 28.5^{o}C. Thus, it is shown that the ointment of the present invention stimulates the blood circulation at the genital portion including penis and scrotum.

### EXPERIMENT 4 : Test for local anesthetic effect

This test was conducted for 20 patients who suffer from premature ejaculation due to sexual nervous hypersensitivity and visit the Yongdong Severance Hospital, Urology Department. The average age of the patients was 39.5 years, the average marriage period was 13.8 years, and the average duration of premature ejaculation was 15.7 years. Among the patients, 15 patients suffered from simple premature ejaculation and 5 patients suffered from premature ejaculation accompanying erection failure. In all the patients the results in physico-chemical examination and neurological examination were normal. The specific test method is described below.

The patients were in a supine position and their eyes were covered up. The examiner randomly stimulated glans penis, scrotum and perineum with a cotton thread so that the patients themselves can identify their stimulating portion. The patients who can accurately identify the touch feeling at all these stimulating portions were selected for this test to record the test results. Using biothesiometry, the electrode was attached to each of index finger, glans penis, scrotum and perineum and then the voltage was slowly increased until the patients feel the vibration sense. The value of meter at which the patients feel the vibration sense was recorded. This test was practiced three times. The lowest value as obtained was selected as the standard. Thereafter, 0.2g of the ointment prepared in EXAMPLE 1 of the present invention was applied to glans penis and then the above test procedure was practiced again to record the result.

According to this test, although the patients can identify the touch feeling at all the portions of index finger, glans penis, scrotum and perineum stimulated by cotton thread before application of the ointment of the present invention, after one hour from application of the ointment of the present invention the touch feeling with cotton thread at glans penis was disappeared in all the patients and no change in any other portions was observed. Before application of the ointment of the present invention, the average vibration sense measured by biothesiometry was 0.04 microns in index finger, 0.05 microns in glans penis, 0.08 microns in scrotum and 0.57 microns in perineum. One hour after application of the ointment of the present invention, the vibration sense of glans penis was determined as 0.10 microns which is double the value measured before the ointment application, and any remarkable change in other portions was not observed.

| Measured portions | Value measured by biothesiometry | |
|---|---|---|
| | Before ointment application | After ointment application |
| index finger | 0.04 microns | 0.04 microns |
| glans penis | 0.05 microns | 0.10 microns |
| scrotum | 0.08 microns | 0.08 microns |
| perineum | 0.57 microns | 0.56 microns |

From the above results, it can be identified that the ointment of the present invention has an activity for blunting topical touch feeling and topical vibration sense, which is believed as being the main mechanism for treatment of preamture ejaculation due to genital hypersensitivity.

From the results of the clinical trials above, it has been identified that since the composition of the present inventions is composed of the galenic components, it has no side effect and that the time for use of the present composition can be freely selected due to its long lasting effect. Furthermore, it is apparent that the composition of the present invention is suitable for satisfactory sexual life since it provides an effect of treating premature ejaculation and further of stimulating blood circulation and therefore is somewhat useful for improving erection failure.

## Claims

1. A topical pharmaceutical composition for prophylaxis and treatment of premature ejaculation comprising alcohol and/or aqueous extracts of raw ginseng radix, angelicae gigantis radix, broomrape, cassiae cortex, asiasari radix and bufonis venenum, with the amounts of said extracts being sufficient to prevent premature ejaculation.

2. The pharmaceutical composition of claim 1 further including at least one component selected from alcohol and/or aqueous extracts of xanthoxyli fructus, cnidium fructus, caryophylli flos and moschus.

3. A pharmaceutical composition for prophylaxis and treatment of premature ejaculation comprising ethanol and/or aqueous extracts of ginseng radix, angelicae gigantis radix, broomrape, cnidium fructus, xanthoxyli fructus, cassiae cortex, caryophylli flos, asiasari radix, bufonis venenum and moschus sufficient to prevent ejaculation before, upon, or shortly after penetration, and a pharmaceutically acceptable carrier, adjuvant or excipient therefor.

4. The pharmaceutical composition of any of claims 1 to 3, wherein the composition is formulated in the form of ointment, suspension, gel, spray or solution.

5. The pharmaceutical composition of any of claims 1 to 4 wherein said alcohol and/or aqueous extracts have been obtained from 60-140 parts by weight of ginseng radix, 60-140 parts by weight of angelicae gigantis radix, 60-140 parts by weight of broomrape, 10-30 parts by weight of cassiae cortex and 10-30 parts by weight of asiasari radix and 15-25 parts by weight of bufonis venenum.

6. The pharmaceutical composition of claim 2 or 5 wherein said alcohol and/or aqueous extracts have been obtained from 40-70 parts by weight of xanthoxyli fructus, 60-140 parts by weight of cnidium fructus, 10-30 parts by weight of caryophylli flos and 5-15 parts by weight of moschus.

7. A process for preparation of pharmaceutical composition useful as an agent for prophylaxis and treatment of premature ejaculation, which comprises forming an alcohol and/or aqueous extract from raw ginseng radix, angelicae gigantis radix, broomrape, cassiae cortex, asiasari radix and bufonis venenum; and
combining said alcohol and aqueous extracts with a pharmaceutically acceptable ointment base.

8. The process of claim 7 including at least one further component selected from alcohol and/or aqueous extracts of xanthoxyli fructus, cnidium fructus, caryophylli flos and moschus.

9. The process of claim 7 wherein said ointment base is a water soluble base.

## Patentansprüche

1. Topisches pharmazeutisches Präparat zur Prophylaxe und Behandlung der vorzeitigen Ejakulation, das alkoholische und/oder wäßrige Extrakte von roher Radix Ginseng, Radix Angelicae gigantis, Orobanche, Cortex Cassiae, Radix Asiasari und Bufotoxin enthält, wobei die Mengen der Extrakte zur Verhinderung der vorzeitigen Ejakulation ausreichen.

2. Pharmazeutisches Präparat nach Anspruch 1, das außerdem wenigstens eine Komponente enthält, ausgewählt aus alkoholischen und/oder wäßrigen Extrakten von Fructus Xanthoxyli, Fructus Cnidii, Flos Caryophylli und Moschus.

3. Pharmazeutisches Präparat zur Prophylaxe und Behandlung der vorzeitigen Ejakulation, das ethanolische und/oder wäßrige Extrakte von Radix Ginseng, Radix Angelicae gigantis, Orobanche, Fructus Cnidii, Fructus Xanthoxyli, Cortex Cassiae, Flos Caryophylli, Radix Asiasari, Bufotoxin und Moschus, die zur Verhinderung der Ejakulation während oder kurz nach der Penetration ausreichen, und einen pharmazeutisch verträglichen Carrier, Hilfsstoff oder Trägerstoff dafür enthält.

4. Pharmazeutisches Präparat nach einem der Ansprüche 1 bis 3, wobei das Präparat in Form von Salbe, Suspension, Gel, Spray oder Lösung formuliert ist.

5. Pharmazeutisches Präparat nach einem der Ansprüche 1 bis 4, wobei die alkoholischen und/oder die wäßrigen Extrakte erhalten wurden aus 60 - 140 Gewichtsteilen Radix Ginseng, 60 - 140 Gewichtsteilen Radix Angelicae gigantis, 60 - 140 Gewichtsteilen Orobanche, 10 - 30 Gewichtsteilen Cortex Cassiae und 10 - 30 Gewichtsteilen Radix Asiasari und 15 - 25 Gewichtsteilen Bufotoxin.

6. Pharmazeutisches Präparat nach Anspruch 2 oder 5, wobei die alkoholischen und/oder die wäßrige Extrakte erhalten wurden aus 40 - 70 Gewichtsteilen Fructus Xanthoxyli , 60 - 140 Gewichtsteilen Fructus Cnidii, 10 - 30 Gewichtsteilen Flos Caryophylli und 5 - 15 Gewichtsteilen Moschus.

7. Verfahren zur Herstellung eines pharmazeutischen Präparates, das als Mittel zur Prophylaxe und Behandlung der vorzeitigen Ejakulation geeignet ist und das folgendes umfaßt: Bilden eines alkoholischen und/oder wäßrigen Extraktes aus roher Radix Ginseng, Radix Angelicae gigantis, Orobanche, Cortex Cassiae, Radix Asiasari und Bufotoxin; und
Zusammenbringen der alkoholischen und wäßrigen Extrakte mit einer pharmazeutisch verträglichen Salbengrundlage.

8. Verfahren nach Anspruch 7, wobei mindestens eine weitere Komponente enthalten ist, ausgewählt aus alkoholischen und/oder wäßrigen Extrakten von Fructus Xanthoxyli, Fructus Cnidii, Flos Caryophylli und Moschus.

9. Verfahren nach Anspruch 7, wobei die Salbengrundlage eine wasserlösliche Grundlage ist.

## Revendications

1. Une composition pharmaceutique topique pour la prophylaxie et le traitement de l'éjaculation précoce comprenant des extraits alcooliques et/ou aqueux de racines de ginseng brut, de racines d'angelicae gigantis, d'orobanche, d'écorces de cassiae, de racines asiasari et bufonis venenum, les quantités desdits extraits étant suffisantes pour empêcher l'éjaculation précoce.

2. La composition pharmaceutique selon la revendication 1 comprenant, en outre, au moins un composant choisi parmi les extraits alcooliques et/ou aqueux de xanthoxyli fructus, cnidium fructus, caryophylli flos et moschus.

3. Une composition pharmaceutique pour la prophylaxie et le traitement de l'éjaculation précoce comprenant des extraits alcooliques et/ou aqueux de racines de ginseng, de racines d'angelicae gigantis, d'orobanche, de cnidium fructus, de xanthoxyli fructus, d'écorces de cassiae, de caryophylli flos, de racines asiasari, de bufonis venenum et moschus en quantité suffisante pour empêcher l'éjaculation avant, lors ou peu de temps après la pénétration et un véhicule acceptable du point de vue pharmaceutique, un adjuvant ou un excipient.

4. La composition pharmaceutique selon l'une quelconque des revendications 1 à 3, dans laquelle la composition est formulée sous la forme d'un onguent, d'une suspension, d'un gel, d'une pulvérisation ou d'une solution.

5. La composition pharmaceutique selon l'une quelconque des revendications 1 à 4, dans laquelle lesdits extraits alcooliques et/ou aqueux ont été obtenus à partir de 60 à 140 parties en poids de racines de ginseng, 60 à 140 parties en poids de racines d'angelicae gigantis, 60 à 140 parties en poids d'orobanche, 10 à 30 parties en poids d'écorces de cassiae et 10 à 30 parties en poids de racines asiasari et 15 à 25 parties en poids de bufonis venenum.

6. La composition pharmaceutique selon la revendication 2 ou 5, dans laquelle lesdits extraits alcooliques et/ou aqueux ont été obtenus à partir de 40-70 parties en poids de xanthoxyli fructus, 60-140 parties en poids de cnidium fructus, 10-30 parties en poids de caryophylli flos et 5-15 parties en poids de moschus.

7. Un procédé pour la préparation d'une composition pharmaceutique utile en tant qu'agent pour la prophylaxie et le traitement de l'éjaculation précoce qui consiste à former un extrait alcoolique et/ou aqueux à partir de racines de ginseng brut, de racines d'angelicae gigantis, d'orobanche, d'écorces de cassiae, de racines asiasari et de bufonis venenum ; et à combiner lesdits extraits alcooliques et aqueux avec une base d'onguent acceptable du point de vue pharmaceutique.

8. Le procédé selon la revendication 7 comprenant au moins un autre composant choisi parmi les extraits alcooliques et/ou aqueux de xanthoxyli fructus, cnidium fructus, caryophylli flos et moschus.

9. Le procédé selon la revendication 7, dans lequel ladite base d'onguent est une base soluble dans l'eau.
